(19) Europäisches Patentamt European Patent Office Office européen des brevets

(11) **EP 1 535 845 B1**

(12) **EUROPEAN PATENT SPECIFICATION**

(45) Date of publication and mention
of the grant of the patent:
**01.04.2009 Bulletin 2009/14**

(21) Application number: **03760159.8**

(22) Date of filing: **17.06.2003**

(51) Int Cl.:
**B65B 55/06** (2006.01)  **B65B 55/14** (2006.01)

(86) International application number:
**PCT/JP2003/007683**

(87) International publication number:
**WO 2003/106270 (24.12.2003 Gazette 2003/52)**

(54) **METHOD OF ASEPTIC FILLING**

VERFAHREN ZUM ASEPTISCHEN ABFÜLLEN

TECHNIQUE DE REMPLISSAGE ASEPTIQUE

(84) Designated Contracting States:
**DE FR GB**

(30) Priority: **17.06.2002 JP 2002175989**

(43) Date of publication of application:
**01.06.2005 Bulletin 2005/22**

(73) Proprietor: **Yoshino Kogyosho Co., Ltd.**
**Tokyo 136-8531 (JP)**

(72) Inventors:
• **IMAI, Toshio**
**c/o Matsudo Factory,**
**Matsudo-shi, Chiba 270-2297 (JP)**

• **KOJIMA, Naoyuki**
**c/o Matusdo Factory,**
**Matsudo-shi, Chiba 270-2297 (JP)**

(74) Representative: **Gray, James**
**Withers & Rogers LLP**
**Goldings House**
**2 Hays Lane**
**London**
**SE1 2HW (GB)**

(56) References cited:
**EP-A- 0 300 978**    **EP-A- 0 624 519**
**GB-A- 978 807**     **JP-A- 5 132 041**
**JP-A- 9 150 896**    **US-A- 4 560 564**

EP 1 535 845 B1

## EP 1 535 845 B1

**Description**

BACKGROUND OF THE INVENTION

Technical Field

The present invention relates to a method for aseptically filling tea or the like beverage in a container.

Prior Art

Bottle-shaped synthetic resin containers (e.g., PET bottles or the like), in which tea or the like beverage is packed as the content, successfully obtained wide support by consumers due to recent tea boom, and its market tends to be progressively expanded. It is important in particular that the contents preserve the natural taste and flavor thereof, and thus desirable to suppress the influence of temperature change of the contents as low as possible. Consequently, so-called aseptic (i.e., abacterial) filling system has been adopted, in which the contents are sterilized and cooled within a short time, and then packed in a sterilized container at normal temperature.

The container used for the aseptic filling is assumed to pack the contents at normal temperature, after having been subjected to sterilization and cooling, and therefore the heat resistance of the container has not been specifically taken into consideration. However, when the contents together with the container are heated, for example, to 65°C by a warmer, hot vender or the like during cold months, such as in winter season, it is likely that the container is deformed by heat thereby degrading the commercial value.

In this respect, trials have been made such as to replace the container used for aseptic filling with a container having improved heat resistance adopted for hot filling, or to increase the wall thickness of the container. However, even in the case of containers with improved heat resistance, the body portion has a heat resistance that is still insufficient for heating by a warmer or the like, while the increased wall thickness of the container is not very desirable from the viewpoint of reduction of resources.

EP 0624519 is directed to a process and apparatus for packaging liquid food products in gable-topped cartons. The food products include high acid liquids such as orange juice.

JP 5132041 discloses an aseptic filling method for a polyethylene tetraphthalate bottle. The bottle is filled at a normal temperature with a pasteurised acidic drink such as fruit juice and hence does not require a subsequent cooling step.

DISCLOSURE OF THE INVENTION

It is therefore an object of the present invention to provide a novel aseptic filling method by which the container filled with the contents can be effectively prevented from deformation upon heating.

To this end, according to the present invention, there is provided a filling method wherein the contents preliminarily sterilized are subjected to aseptically filled in a synthetic resin container subjected to rinsing and sterilization, and wherein the contents are filled under a temperature which is higher than normal temperature, but lower than a hot filling temperature intended for sterilization effect, i.e., a temperature for expressing sterilization effect (about 80°C or higher). Here, the term "normal temperature" generally denotes a predetermined standard temperature condition as prescribed in JIS Z 8703 or corresponding ISO 554: 1976: "*Standard Atmospheres For Conditioning and/or Testing*", i.e., a temperature at about 20°C, and this applies to the present application as well.

However, when a cooling step for cooling the filled contents to normal temperature is added as the final step of the aseptic filling method, the contents upon filling may be at a temperature achieved by heating by means of a warmer or the like heater, for example, at a temperature within a range of about 50°C to 60°C.

According to the present invention there is provided a method for aseptically filling a synthetic resin container and subsequently heating the filled container before vending or serving, the filled container being heatable to a temperature of between 50°C and 60°C without degrading the appearance and shape of the container, the method comprising the steps of:

    rinsing and sterilizing a synthetic container;
    preliminary sterilizing of a content for a container;
    filling the content in the synthetic resin container under a temperature within a range of 50°C and 60°C;
    cooling the content to normal temperature; and
    heating the filled container to a temperature of 50°C and 60°C before vending or serving.

## BEST MODE FOR CARRYING OUT THE INVENTION

The aseptic filling method according to the present invention will be more fully described below.

Although various systems have been proposed with respect to aseptic filling, one example will be explained below. There is known a system wherein a container sterilizing section is combined with an aseptic filling section. In this case, the inside of a bottle, which has been produced by blow-molding process or the like, is rinsed with warm water at the container sterilizing section, the outside of the bottle is then rinsed with a sterilizing solution using the mouth portion of the bottle as a target, the inside of the bottle is sterilized by being fully packed with a sterilizing solution, the sterilizing solution is discharged and aseptic air flushing is carried out with the mouth portion of the bottle directed downwards. At the subsequent aseptic filling section, the contents are aseptically filled in the bottle to which the aseptic air flushing has been carried out, followed by capping treatment.

It has been conventionally considered that, when liquid is packed in a container through the above-described aseptic filling method and such container is heated by a warmer or the like, the shape deformation of the container by thermal influence upon heating would be inevitable. However, according to the present invention, the thermal influence with respect to the container upon heating can be effectively mitigated by maintaining, upon aseptic filling, the contents at a temperature that is higher than normal temperature, but lower than a temperature expressing the sterilization effect. By this, the shape and appearance of the container (hence, the commercial value) are positively prevented from degradation.

The reason why the container is prevented from deformation in shape and degradation in appearance by maintaining the content at a temperature higher than normal temperature and carrying out the aseptic filling will be explained below. Thus, when the contents maintained at a temperature higher than normal temperature are filled, the final inner pressure in the container is lower as compared to the case of carrying out the aseptic filling under normal temperature condition. Therefore, even if the container is heated in such conditions, it would be possible to suppress increase in the inner pressure upon heating, by the decreased amount of the inner pressure.

The above-mentioned mechanism will be more fully clarified below. When 280 ml of water is aseptically packed at normal temperature (20°C) and at 40°C in PET bottles having a full volume of 290 ml (residual space in the bottle is 10 ml), the inner pressure of the bottle is as below. Further, upon consideration of the influence of the inner pressure to the bottle, it is assumed that the container is rigid for ignoring the mitigation of the pressure caused by deformation of the container, when the level of increase in the inner pressure is calculated, and the change of the volume caused by thermal expansion of the container is also assumed to be negligible.

First of all, 280 ml of water at 20°C changes its volume to 281.69 ml at 40°C and 285.04 ml at 65°C caused by thermal expansion. It is assumed that the gas in the space of the container follows the state equation of ideal gas: PV = nRT. Since the water vapor pressure is 2.338 kPa at 20°C, 7.377 kPa at 40°C and 25.014 kPa at 65 °C, the difference in water vapor pressure upon heating from 20°C to 65°C is about 23 kPa (25.014 - 2.338 = 22.676), and the difference in water vapor pressure upon heating from 40°C to 65°C is about 18 kPa (25.014 - 7.377 = 17.637).

When the container is packed at 20°C and then heated to 65°C, namely, when it is aseptically packed according to conventional process and heated, the pressure in the space within the container is determined by:

$$PV = nRT$$

$$P'V' = nRT'.$$

Here, n and R are constants, P = 101.325 kPa (pressure at filling = atmospheric pressure), V = 10 ml (the residual space volume within container upon filling), T = 293 K (absolute temperature at filling 20 + 273), P' = pressure of space in container upon heating, V' = 290 - 285.04 = 4.96 ml (the residual space volume within the container upon heating), T' = 338 K (the absolute temperature upon heating; 65 + 273). In this instance, since PV/T = P'V'/T', the pressure P' in the space within the container upon heating is:

$$P' = PVT'/TV'$$
$$= (101.325 \times 10 \times 338)/(293 \times 4.96)$$
$$= 236 \text{ kPa}$$

Further, when 23 kPa of the difference in water vapor pressure is added, the final inner pressure (absolute pressure) is 236 + 23 = 259 kPa (158 kPa by gauge pressure).

On the other hand, when the container is packed at 40˚C and heated to 65˚C, the pressure in the space within the container is similarly determined in the manner as described above. Here, P = 101.325 kPa (the pressure upon filling = atmospheric pressure), V = 8.31 ml (the residual space volume within the container upon filling 280 ml of water at 20˚C and maintained at 40˚C), T = 313 K (the absolute temperature upon filling 40 + 273), P' = the pressure in the space of the container at heating, V' = 290 - 285.04 = 4.96 ml (the residual space volume within the container upon heating), T' = 338 K (the absolute temperature upon heating 65 + 273). Accordingly, the pressure P' in the space of the container upon heating is

$$P' = PVT'/TV'$$
$$= (101.325 \times 8.31 \times 338)/(313 \times 4.96)$$
$$= 183 \text{ kPa}$$

When 18 kPa of the difference in water vapor pressure is added, the final inner pressure (absolute pressure) is 183 + 18 = 201 kPa (100 kPa by gauge pressure).

As described above, the difference of the inner pressure between the filling at normal temperature (20˚C) and the filling at 40˚C is 158 - 100 = 58 kPa by gauge pressure, and the difference of the inner pressure is considered to be a factor capable of suppressing the shape deformation of the container when the container subjected to aseptic filling is heated. As a matter of fact, since the inner pressure is mitigated by the elastic deformation of the container, it becomes further low pressure.

In general, a warmer or the like heater is used so as to maintain the content in the container at a temperature of about 50˚C to 60˚C. Accordingly, from the viewpoint of suppressing increase in the inner pressure of the container upon heating, it would be best to pack the content under the similar temperature as the heating temperature at the aseptic filling. In this case, it is preferred to add a cooling step for cooling the content to normal temperature as the final step of the aseptic filling process. Alternatively, when the installation of a cooling device and extension of the cooling line are undesirable, it is preferred that the upper limit temperature of the content is kept at about 30˚C to 40˚C during the aseptic filling, i.e., at the temperature capable of suppressing deformation of the container without requiring the cooling step.

Verification Experiments

Circular-sectioned heat resistive PET bottles with a shrink label having a filling volume of 280 ml (the amount of the resin used: 26 g), and square-sectioned highly heat resistive PET bottles with a shrink label having a filling volume of 350 ml (the amount of the resin used: 26 g) were prepared, water at 20˚C and water at 40˚C were charged in the respective containers, caps were fastened and then the changes in shape and appearance were observed when they were heated in a water bath, i.e., immersed in respective isothermal water vessels at 60˚C, 65˚C, 70˚C, 75˚C and 80˚C for 1 hour.

The result of the experiments is shown in Table 1. Further, the appearances of the containers were evaluated by judging according to the situations of the convex portions at the bottom portions and panel portions (body portions) of the containers when they are cooled to normal temperature. Symbol "x" indicates a case of generating projections on the convex portions of bottom portions and the panel portions after cooling, symbol "Δ" indicates a case of generating projections on the slight convex portions of bottom portions and the panel portions after cooling, symbol "○" indicates a case that practically negligible slight convexes were generated on the bottom portions, and symbol "◎" indicates a case of generating no problems at all.

Table 1

| Test temperature (˚C) | Circular-sectioned heat resistive PET bottle of 280 ml | | Square-sectioned heat resistive PET bottle of 350 ml | |
|---|---|---|---|---|
| | Filling at 20˚C | Filling at 40˚C | Filling at 20˚C | Filling at 40˚C |
| 85 | × | × | × | × |
| 75 | × | × | Δ | ○ |

(continued)

| Test temperature (˚C) | Circular-sectioned heat resistive PET bottle of 280 ml | | Square-sectioned heat resistive PET bottle of 350 ml | |
|---|---|---|---|---|
| | Filling at 20˚C | Filling at 40˚C | Filling at 20˚C | Filling at 40˚C |
| 70 | - | - | ○ | ◎ |
| 65 | ○ | ◎ | ◎ | ◎ |
| 60 | ◎ | ◎ | - | - |

As clearly shown in Table 1, it could be confirmed that the change in shape of the container is significantly suppressed by maintaining the contents at a higher temperature than normal temperature upon aseptic filling, the circular-sectioned heat resistive PET bottle can withstand the heating to about 60˚C and the square-sectioned heat resistive PET bottle can withstand the heating to about 65˚C.

It will be appreciated from the foregoing detailed description that, according to the present invention, since the aseptic filling is carried out by setting the temperature of the contents at a level higher than normal temperature, the temperature upon heating approaches to the temperature at filling as compared to the case of filling at normal temperature and the increase in the inner pressure of the container upon heating is mitigated. Therefore, even if the contents are heated together with the container, it is possible to effectively and significantly suppress the influence degrading the shape and appearance of the container.

While the present invention has been described above with reference to the preferred embodiments, it is needless to say that various modifications from the embodiments described above may be adopted without deviating from the scope of the invention.

**Claims**

1.  A method for aseptically filling a synthetic resin container and subsequently heating the filled container before vending or serving, the filled container being heatable to a temperature of between 50˚C and 60˚C without degrading the appearance and shape of the container, the method comprising the steps of:

    rinsing and sterilizing a synthetic container;
    preliminary sterilizing of a content for a container;
    filling the content in the synthetic resin container under a temperature within a range of 50˚C and 60˚C;
    cooling the content to normal temperature; and
    heating the filled container to a temperature of between 50˚C and 60˚C before vending or serving.

**Patentansprüche**

1.  Verfahren zum aseptischen Abfüllen eines Kunstharzbehälters und zum anschließenden Erwärmen des abgefüllten Behälters vor dessen Verkauf oder Servieren, wobei der abgefüllte Behälter auf eine Temperatur zwischen 50˚C und 60˚C erwärmbar ist, ohne dass das Aussehen und die Form des Behälters verschlechtert werden, wobei das Verfahren die folgenden Schritte umfasst:

    Spülen und Sterilisieren eines synthetischen Behälters;
    Vorsterilisieren eines Inhalts für einen Behälter;
    Einfüllen des Inhalts in den Kunstharzbehälter unter einer Temperatur in einem Bereich von 50˚C und 60˚C;
    Kühlen des Inhalts auf Normaltemperatur und
    Erwärmen des abgefüllten Behälters auf eine Temperatur zwischen 50˚C und 60˚C vor dessen Verkauf oder Servieren.

**Revendications**

1.  Procédé de remplissage aseptique d'un récipient en résine synthétique puis de chauffage du récipient rempli avant la vente ou le service, le récipient rempli pouvant être chauffé à une température entre 50˚C et 60˚C sans détériorer l'aspect et la forme du récipient, le procédé comprenant les étapes de :

rincer et stériliser un récipient synthétique ;
stériliser de manière préliminaire un contenu pour un récipient ;
introduire le contenu dans le récipient en résine synthétique à une température dans une plage de 50°C à 60°C ;
refroidir le contenu à la température normale ; et
chauffer le récipient rempli à une température entre 50°C et 60°C avant la vente ou le service.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0624519 A

- JP 5132041 B